# EUROPÄISCHE PATENTANMELDUNG

(11) **EP 0 759 483 A1**
(43) Veröffentlichungstag der Anmeldung: **26.02.1997**
(21) Anmeldenummer: 95112992.3
(22) Anmeldetag: 18.08.1995
(51) Int. Cl.: D01G 31/00, G01N 33/36, G01N 21/89

(54) **Verfahren zum Erfassen von Fremdfasern an einer Öffnungsmaschine**

(71) Anmelder: Hergeth, Hubert, Dipl. Ing.-Dipl. Wirt. Ing., 4731 Eynatten (BE)
(72) Erfinder: Hergeth, Hubert, Dipl. Ing.-Dipl. Wirt. Ing., 4731 Eynatten (BE)

(57) **Zusammenfassung**

In einer Öffnungsmaschine für Fasermaterial werden die Fasern mittels Sensoren kontrolliert und andersfarbige Bestandteile detektiert. Die Lichtstrahlen von einem Beobachtungsfenster zu den Sensoren werden umgelenkt, um eine platzgünstige Unterbringung in bestehenden Öffnungskonstruktionen zu ermöglichen.

## Beschreibung

Fremdfasern gelangen als größere Gewebeteile vor der Baumwollernte zu den Baumwollflocken. In der Egrenieranstalt werden diese Gewebeteile oder Folien stark zerkleinert und mit den Baumwollflocken vermengt. Die Öffnung der Faserballen mit Ballenfräsen sowie der Transport der Fasern in Rohrleitungen, führt zu einer weiteren "Verpackung" der Fasern im Fasermaterial. Um kleine, andersfarbige Bestandteile in einem kontinuierlichen Flockentransport zu erkennen, ist es notwendig, die Fasern weiter aufzulösen, um so die zu betrachtende Oberflache zu vergrößern und zu verdünnen.

Die DE 195 18 783.0 schlägt deshalb vor, die Fasern an einer Öffnungsmaschine zu kontrollieren. Die preiswertesten Sensoren zur Kontrolle stellen sogenannte CCD-Zeilenkameras dar. Auf einem Chip sind in einer Reihe lichtempfindliche Zellen angebracht, die Informationen über die Lichtintensität, die die einzelnen Zellen trifft, liefert. Werden mehrere Zeilen mit unterschiedlichen Farbfiltern versehen und die Meßresultate kombiniert, kann eine Farbauswertung erfolgen. Eine Zeile kann aus mehr als 1000 Elementen bestehen. Die Zeile auf dem Chip ist nur bis etwa 4 cm lang. Über ein Objektiv werden die Lichtstrahlen auf die Zeile geleitet. Ein 1 m breiter Kontrollkorridor auf der Öffnungswalze kann so mit einem 4 cm breiten CCD-Chip in 1000 mm Auflösung kontrolliert werden.

Es ist von großem Vorteil, wenn der Abstand zwischen CCD-Sensor, Objektiv und Beobachtungsfenster groß ist. Dieser Abstand könnte etwa 1-2 mal der Breite des Beobachtungskorridors, der etwa der Walzenbreite entspricht, sein. Ein enger Abstand bewirkt eine starke Reflektion an dem Beobachtungsfenster, sodaß die Intensität der Lichtstrahlen nachläßt. Es müssen auch Objektive, die stark verzerrenden "Fischaugen", eingesetzt werden. Eine andere Möglichkeit, den Abstand zu verringern, besteht in dem Einsatz mehrerer paralleler, über die Korridorbreite verteilt, montierter Sensoren. Die Lösung ist jedoch kostenintensiver. Es ist schwierig, an bestehenden Konstruktionen von Öffnungsmaschinen eine Möglichkeit zu finden, einen über 1 m langen Beobachtungskanal auf die Öffnungswalze zu richten. Es ist vorteilhaft, das Beobachtungsfenster im Faserfluß nach mindestens einem Reinigungselement (Messer) an der Öffnungswalze zu palzieren, sodaß Samenkörner vor der Erkennung ausgeschieden werden und so keine Fehlauslösung als Fremdfarbe verursachen.

Aufgabe der Erfindung ist es, eine Möglichkeit zu finden, eine preiswerte, hochauflösende Fremdfasererkennung an einer Öffnungsmaschine zu realisieren. Dies geschieht erfindungsgemäß durch die Umlenkung der Lichtstrahlen in einem Schacht, sodaß die, für einen kleinen Winkel notwendige Länge, zwischen Beobachtungsfenster an der Walze und Objektiv vor dem CCD- Chip, an einer Stelle der Öffnungsmaschine sich erstrecken kann, wo Raum vorhanden ist. In der Praxis wird die vorhandene Öffnungsmaschine auf einen Sockel gehoben, der sich über die Grundflache der Öffnungsmaschine erstreckt. In dem Sockel ist Platz für die CCD-Kameras und die Beleuchtungseinheiten, sowie eine lange Strecke bis zu einem Spiegel, der die Lichtstrahlen umlenkt. Der Strahlengang trifft durch einen schmalen Schacht auf das Beobachtungsfenster. Der schmale Schacht, vom Sockel zum Beobachtungsfenster, ist einstellbar. Durch die Umlenkung des Strahles ist es möglich, einfache CCD - Sensoren in einem Sockel unterhalb einer bestehenden Öffnungsmaschinenkonstruktion optimal unterzubringen. Die vorgeschlagene Lösung erlaubt es, eine Fremdfaserdetektion mit geringeren Mitteln und besserem Detektionsergebnis als bisher bekannt zu realisieren.

Das Verfahren wird im folgenden anhand einer gebräuchlichen Öffnungsmaschinenkonstruktion beschrieben. Die Öffnungsmaschine besteht aus einem Gehäuse "1" mit mindestens einer Öffnungswalze "4", auf deren Umfang Stifte "6" oder andere Mitnahmeeinrichtungen angebracht sind. Die Fasern werden über ein Zufuhrtransportband "2" einem Einzugswalzenpaar "3" zugeführt. Die Mitnahmeeinrichtung der Walze erfassen die Fasern und führen sie an Reinigungselementen "5" vorbei, an denen Samenkörner, Steine und Stengelreste abgestreift werden. Die Fasern verlassen die Reinigungsmaschine durch einen Absaugkanal "9". Unter der Reinigungsmaschine ist ein Sockel "13" angebracht. An der Stirnseite des Sockels sind mindestens eine CCD- Kamera "12" mit Objektiv befestigt und mehrere Beleuchtungseinheiten "11". Durch einen Spiegel "10" der verstellbar angebracht ist, werden die Lichtstrahlen umgelenkt, sodaß mit dem im Sockel angebrachten Kameras auf das Fenster "7" des Beobachtungskorridors geblickt werden kann. Ein kleiner Schacht "14" verbindet den Sockel mittels elasticher Verbindungen "8" mit einem Beobachtungsfenster.

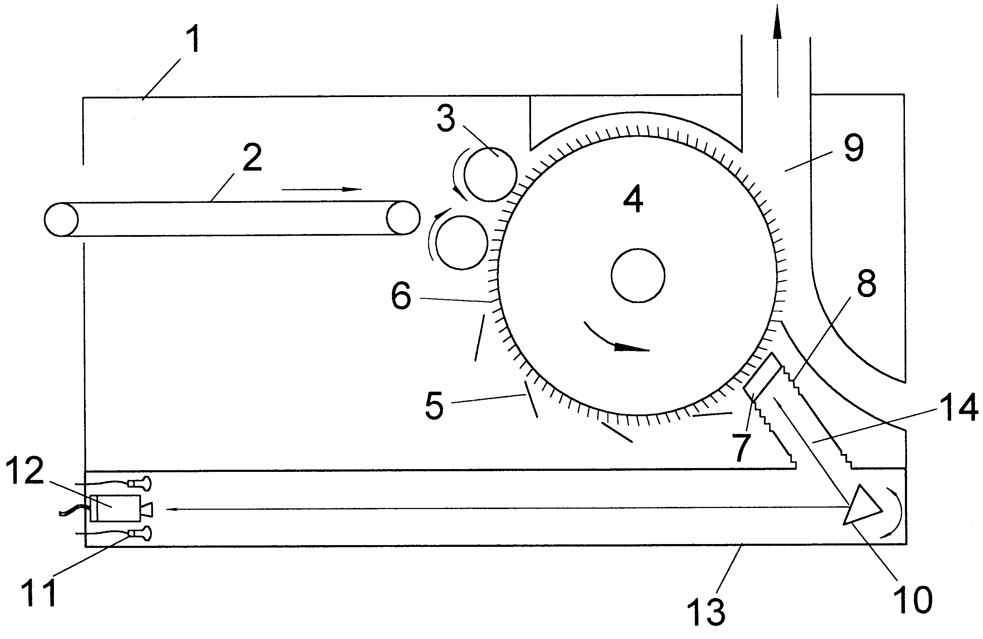

## Patentansprüche

1. Verfahren zum Erfassen von Folien, Geweben und Fasern, die eine andere Farbe aufweisen als die normal verarbeiteten Fasern in einer Öffnungsmaschine einer Faserverarbeitungslinie, dadurch gekennzeichnet, daß die Lichtstrahlen zwischen den zu erkennenden Teilen und den Sensoren zu deren Erfassung durch mindestens einen Spiegel oder mindestens ein Prisma umgelenkt werden.

2. Verfahren nach Anspruch 1 dadurch gekennzeichnet, daß die Lichtstrahlen für mehrere Sensoren durch den gleichen Spiegel oder das gleiche Prisma umgelenkt werden.

3. Verfahren nach einem der Ansprüche 1 und 2 dadurch gekennzeichnet, daß die umgelenkten Lichtstrahlen auf eine Öffnungswalze, eine Transportwalze, ein Transportband oder auf einen Transportluftstrom treffen.

4. Verfahren nach einem der Ansprüche 1-3dadurch gekennzeichnet, daß die Umlenkspiegel oder Prismen verstellbar im Sockel montiert sind.

5. Verfahren nach einem der Ansprüche 1-4 dadurch gekennzeichnet, daß das Beleuchtungslicht für den Beobachtungskorridor ebenfalls über Spiegel und Prismen umgelenkt wird.

6. Verfahren nach einem der Ansprüche 1-5 dadurch gekennzeichnet, daß die Umlenkung der Lichtstrahlen mehr als 30° beträgt.

7. Verfahren nach einem der Ansprüche 1-6 dadurch gekennzeichnet, daß die CCD-Kameras unterhalb der Achse der Öffnungswalze angebracht sind.

8. Verfahren nach einem der Ansprüche 1-7 dadurch gekennzeichnet, daß als Sensoren Photoelemente von Zeilenkameras oder Matrixkameras verwendet werden.

9. Verfahren nach einem der Ansprüchen 1-8 dadurch gekennzeichnet, daß die Verbindung zwischen Betrachtungsfenster und Betrachtungskanal elastisch ausgeführt ist.

10. Verfahren nach einem der Ansprüche 1-9 dadurch gekennzeichnet, daß die Lichtstrahlen durch die Umlenkvorrichtung auf einen Korridor der Öffnungswalze gerichtet werden, der sich, in Materialflußrichtung gesehen, hinter dem ersten Messer befindet.
